(19) Europäisches Patentamt
European Patent Office
Office européen des brevets

(11) Veröffentlichungsnummer: **0 182 373 B1**

(12) **EUROPÄISCHE PATENTSCHRIFT**

(45) Veröffentlichungstag der Patentschrift: **27.03.91**

(51) Int. Cl.⁵: **G01N 33/52**, G01N 33/86, C12Q 1/56

(21) Anmeldenummer: **85114744.7**

(22) Anmeldetag: **19.11.85**

(54) **Gerinnungstest auf Teststreifen.**

(30) Priorität: **19.11.84 DE 3442271**
**08.05.85 DE 3516579**

(43) Veröffentlichungstag der Anmeldung:
**28.05.86 Patentblatt 86/22**

(45) Bekanntmachung des Hinweises auf die Patenterteilung:
**27.03.91 Patentblatt 91/13**

(84) Benannte Vertragsstaaten:
**AT BE CH DE FR GB IT LI LU NL SE**

(56) Entgegenhaltungen:
**EP-A- 0 103 247**
**EP-A- 0 111 836**

**ANALYTICAL CHEMISTRY, Band 55, Nr. 4, April 1983, Seiten 498A-514A, Washington DC, US; B. WALTER "Dry reagent chemistries in clinical analysis"**

(73) Patentinhaber: **BOEHRINGER MANNHEIM GMBH**
**Sandhofer Strasse 116**
**W-6800 Mannheim 31(DE)**

(72) Erfinder: **Bartl, Knut**
**Am Westend 6**
**W-8121 Wilzhofen(DE)**
Erfinder: **Becker, Udo**
**Birkenweg 2**
**W-3550 Marburg 1(DE)**
Erfinder: **Lill, Helmut**
**Zugspitzstr. 24**
**W-8121 Wielenbach(DE)**
Erfinder: **Wielinger, Hans**
**Im Langgewann 7**
**W-6940 Weinheim(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**W-8000 München 86(DE)**

**EP 0 182 373 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CLINICAL CHEMISTRY, Band 29, Nr. 9, September 1983, Seiten 1641-1658, Washington, US; J. FAREED et al.: "Molecular markers of hemostatic disorders: Implications in the diagnosis and therapeutic management of thrombotic and bleeding disorders"**

**CLINICAL CHEMISTRY, Band 29, Nr. 2, Februar 1983, Seiten 225-236, Washington, US; J. FAREED et al.: "Diagnostic efficacy of newer synthetic-substrates methods for assessing coagulation variables: A critical overview"**

**Beschreibung**

Für die klinische Diagnose, Therapie und Prophylaxe haben Methoden zur Bestimmung des Blutgerinnungssystems, insbesondere was die Funktionsfähigkeit dieses Systems und die Aufdeckung etwa vorhandener Mängel desselben betrifft, bereits jetzt eine große Bedeutung erlangt, die aber noch laufend steigt. Infolgedessen steigt auch das Bedürfnis nach einfachen, ohne großen Aufwand durchführbaren Methoden für derartige Gerinnungstests.

Unter den Methoden zur Bestimmung klinisch relevanter chemischer Parameter haben die auf Teststreifen durchgeführten Bestimmungsmethoden große Verbreitung erlangt, da sie in der Regel ohne oder mit nur geringem apparativem Aufwand rasch eine Bestimmung durchzuführen erlauben. Derartige Teststreifenmethoden haben mittlerweile einen hohen Genauigkeitsgrad erreicht und ermöglichen daher auch quantitative Bestimmungen mit großer Zuverlässigkeit.

Bisher ist es jedoch noch nicht gelungen, derartige Teststreifenmethoden auch zur Bestimmung des Blutgerinnungssystems anzuwenden. Beim Gerinnungssystem handelt es sich um ein Multifaktorensystem, welches außerordentlich schwierig genau zu definieren ist. Hinzu kommt weiter, daß die Gerinnungskaskade von Oberflächenkräften sehr stark beeinflußt wird, wie beispielsweise in British Medical Bulletin (1978), Vol. 34, 107-112 oder Blood, Vol. 59 (1982), 38-42, beschrieben wird. Hinzu kommt, daß in jedem Plasma Thrombozyten vorhanden sind, die durch feste Oberflächen aktiviert werden und in aktiviertem Zustand ihrerseits wiederum die Gerinnungskaskade beeinflussen und damit verfälschen.

Aus EP-A 111 836 ist ein Verfahren zur Bestimmung von Heparin in Blutplasma bekannt, bei dem auf einem Träger ein Thrombin und ein mit Thrombin reagierendes Substrat vorhanden sind. Heparin wird dabei bestimmt, indem man die interessierende Probe mit Anti-Thrombin III, Thrombin und dem Thrombin-sensitiven Substrat umsetzt. Da Heparin ein Inhibitor der Bildung des Komplexes aus Thrombin und Anti-Thrombin III ist, kann umso mehr Thrombin mit dem Thrombin-Substrat reagieren, je mehr Heparin vorhanden ist.

Überraschenderweise wurde nunmehr gefunden, daß es möglich ist, auch Blutgerinnungstests, bei denen die Gerinnungskaskade mindestens teilweise abläuft, auf Teststreifen durchzuführen.

Dies gelingt erfindungsgemäß mit einem Trockenreagenz zur Durchführung von Quick-Tests, PTT-Tests, zur Bestimmung von Prothrombin, Faktor X, Faktor VIII, Faktor VII und Faktor IX, gekennzeichnet durch ein Trägermaterial, welches ein chromophores Substrat einer Protease des Blutgerinnungs-Systems, mit mindestens einem Faktor und/oder Cofaktor des Blutgerinnungs-Systems und mit Puffersubstanz enthält.

Die Auswertung der erfindungsgemäßen Teststreifen kann an sich nach allen hierfür bekannten Methoden erfolgen, beispielsweise mit Hilfe einer Farbskala durch Vergleich. Bevorzugt wird jedoch eine Remissionsphotometrie wegen der höheren Genauigkeit. Bei Verwendung einer Remissionsphotometrie kann außer der Endpunktmessung auch eine kinetische Messung durchgeführt werden. Ebenfalls können die Tests mit und ohne Vorinkubation ausgeführt werden.

Das erfindungsgemäße Trockenreagenz eignet sich je nach der speziellen Zusammensetzung für die Durchführung des Quick-Tests, des PTT-Tests (partielle Thromboplastinzeit), zur Bestimmung von Prothrombin, Faktor X, Faktor VIII, Faktor VII und Faktor IX. Wird das erfindungsgemäße Trockenreagenz zur Durchführung des Quick-Tests verwendet, so enthält es Thromboplastin, ein chromophores Thrombinsubstrat und $Ca^{2+}$-Ionen.

Wird das erfindungsgemäße Trockenreagenz für die Bestimmung von Prothrombin verwendet, so enthält es chromophores Thrombinsubstrat, Faktor Xa und als Cofaktoren Faktor V, $Ca^{2+}$ und Phospholipid.

Wird das erfindungsgemäße Trockenreagenz für die Bestimmung von Faktor X verwendet, so enthält es das Gift der Russel's Viper (RUV) bzw. den reinen oder teilweise aufgereinigten Faktor X-Aktivator aus diesem Gift, $Ca^{2+}$ und ein chromophores Faktor Xa-Substrat.

Wird das erfindungsgemäße Trockenreagenz für die Bestimmung von Faktor VIII verwendet, so enthält es Faktor IXa sowie Spuren von Thrombin, $Ca^{2+}$ , Phospholipid und ein chromophores Faktor Xa Substrat. Es ist auch möglich, statt des Faktor Xa-Substrats ein chromophores Thrombinsubstrat zu verwenden.

Wird das erfindungsgemäße Trockenreagenz für die Bestimmung von Faktor VII verwendet, so enthält es Thromboplastin, $Ca^{2+}$ und ein chromophores Faktor Xa-Substrat.

Wird das erfindungsgemäße Trockenreagenz für die Bestimmung von Faktor IX verwendet, so enthält es aktivierte Kontaktfaktoren mit einem ausreichenden Gehalt an Faktor XIa bzw. einen Faktor XIa, $Ca^{2+}$ und chromophores Faktor IXa-Substrat. Alternativ kann das Reagenz statt eines Faktor IXa-Substrats zusätzlich Phospholipid, Faktor VIII, Spuren von Thrombin und ein chromophores Faktor Xa-Substrat enthalten.

Für die Durchführung des PTT-Tests enthält das erfindungsgemäße Reagenz partielles Thromboplastin,

Kontaktaktivatoren, ein chromophores Thrombinsubstrat, Phospholipid und $Ca^{2+}$. Als Kontaktaktivator verwendet man vorzugsweise Ellagsäure.

Das erfindungsgemäße Trockenreagenz kann im Prinzip aus einem einzigen Trägermaterial (Reaktionsmatrix) bestehen, welches Substrat, Blutgerinnungsfaktor bzw. -cofaktor und Puffersubstanz enthält. Vorzugsweise enthält das Trockenreagenz jedoch noch ein zweites Trägermaterial mit einem Oxidationsmittel (Oxidationsmatrix). In diesem Falle enthält das erste Trägermaterial ein mit dem Chromophor des chromophoren Substrats in Gegenwart des Oxidationsmittels des zweiten Trägermaterials farbbildendes Anilin- oder Phenolderivat.

Prinzipiell kann das erfindungsgemäße Trockenreagenz ein beliebiges chromophores Substrat einer Protease des Blutgerinnungssystems enthalten, vorausgesetzt, dieses Substrat verändert in Gegenwart des Trägermaterials den Ablauf der Gerinnungskaskade nicht. Als gut geeignet erwiesen sich im Rahmen der Erfindung als chromophore Substrate Verbindungen der allgemeinen Formel I

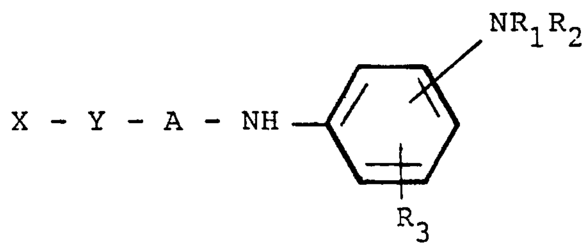

in der

| | |
|---|---|
| A | die Aminosäure Arginin oder Lysin, |
| X | eine N-terminale Aminosäure-Schutzgruppe, |
| Y | eine Einfachbindung oder eine aus 1 bis 3 Aminosäuren bestehende Kette, |
| $NR_1R_2$ | eine in o- oder p-Stellung stehende Gruppe mit $R_1$ oder $R_2$ unabhängig voneinander jeweils Wasserstoff oder Alkyl mit 1 bis 3 C-Atomen oder $NO_2$ und |
| $R_3$ | ein Wasserstoffatom, eine Carboxylester- oder Carboxylamidogruppe, ein Halogenatom, eine Nitrogruppe oder eine Alkylgruppe mit 1 bis 3 C-Atomen |

bedeutet.

Besonders bevorzugt wird im Rahmen der Erfindung ein chromophores Substrat, in dem X-Y-A die Bedeutung Tos-Gly-Pro-Arg hat.

Als farbbildendes Anilin- oder Phenolderivat können die dem Fachmann für diesen Zweck bekannten Verbindungen verwendet werden. Bevorzugt werden N-Methylanthranilsäure, Dimethylanthranilsäure, N-Ethyl-n(3'-sulfobenzol)-anilin und 2,3-Xylenol.

Im Fall der bevorzugten Ausführungsform der Erfindung, welche ein zweites mit einem Oxidationsmittel imprägniertes saugfähiges Trägermaterial enthält (Oxidationsmatrix), ist das erste saugfähige Trägermaterial (Reaktionsmatrix) vorzugsweise mit Tos-Gly-Pro-Arg-p-Phenylendiamin als chromophorem Substrat, mit N-Methylanthranilsäure als farbbildendem Anilinderivat imprägniert und das zweite saugfähige Trägermaterial enthält das Oxidationsmittel $K_3(Fe(CN)_6)$.

Das erfindungsgemäße Reagenz eignet sich für die Bestimmung mit Plasma oder mit Vollblut. Falls die Bestimmung mit Vollblut erfolgen soll ist es zweckmäßig, zusätzlich ein drittes saugfähiges Trägermaterial (Auftragsmatrix) vorzusehen, auf welches die Blutprobe aufgebracht wird. Außerdem wird zweckmäßig noch wenigstens ein Faservlies, welches zwischen dem dritten und dem ersten Trägermaterial angeordnet ist und als Trenn- und Transportmatrix dient, angeordnet. Ein derartiges, als Trenn- oder Transportmatrix dienendes Faservlies kann auch zwischen dem ersten und zweiten Trägermaterial angeordnet sein. Zum Aufbau und zur Wirkung solcher Teste vergleiche DE-A 30 29 579.5, auf die hiermit Bezug genommen wird.

Als Trägermaterial für die Reaktionsmatrix kann im Rahmen der Erfindung ein saugfähiges oder quellfähiges oder lösliches filmbildendes Trägermaterial, z. B. ein für Teststreifen bekanntes Trägermaterial wie Papier und ähnliche Vliesmaterialien, beispielsweise Teebeutelpapier und dergleichen, verwendet werden.

Als Trägermaterialien für die Reaktionsmatrix kommen ebenso quellfähige Substanzen, z. B. Gelatine- oder Zellulosefilme, in Frage, welche durch Einlagerung von Pigmenten durchlässiger gemacht werden können. Das Trägermaterial kann jedoch auch aus einem wasserlöslichen, filmbildenden Polymeren bestehen, in welches die Reagenzien eingelagert sind und das sich in der Substratlösung ganz oder teilweise auflöst, so daß die Reagenzien zur Wirkung kommen. Der reagenzienhaltige Film ist entweder, wie nachstehend beschrieben, auf einer Basisfolie oder auf die Oberfläche der Trenn- oder Transportmatrix

oder das zweite Trägermaterial aufgebracht.

Diese Polymere müssen bei Temperaturen von 20 bis 50°C in Wasser löslich sein und aus ihnen müssen sich Filme herstellen lassen, die wasserquellbar oder wasserlöslich sind. Als Polymere können z. B. eingesetzt werden: Cellulosederivate wie: Methylcellulosen, Methylhydroxyethylcellulosen, Hydroxyethylcellulosen, Hydroxypropylcellulosen, Hydroxypropylmethylcellulosen. Teil- und vollverseifte Polyvinylacetate, Polyvinylpyrrolidone, Polyethylenoxide, Gelatinen, Polyxanthane, Polyacylamide usw.

Als Trägermaterial für die Oxidationsmatrix können die für Teststreifen bekannten Trägermaterialien wie Papier-, Glas- oder Kunststoffvliese, Netze und Gewebe aus Fasermaterial oder saugfähige, poröse Filme oder Gele Verwendung finden. Als Trenn- und Transportmatrix dienende Faservliese werden zweckmäßig aus einem inerten, nicht für sich saugfähigen Fasermaterial, vorzugsweise aus Glasfaser, Kunststoffaser, anderen künstlichen oder natürlichen Mineralfasern und dergleichen bestehen.

Ferner kann das erfindungsgemäße Reagenz auch eine Basisfolie und/oder eine Abdeckfolie, die vorzugsweise transparent ist, sowie gegebenenfalls ein oder mehrere Abstandsblöcke enthalten. Solche Basis- und Abdeckfolien und Abstandsblöcke sind beispielsweise in der DE-A 30 29 579.5 beschrieben.

Die Herstellung des erfindungsgemäßen Reagenz erfolgt so, daß zuerst eine Tränklösung geeigneter Zusammensetzung hergestellt und das saugfähige Trägermaterial dann mit derselben imprägniert und getrocknet wird. Die Tränklösungen enthalten außer den oben schon erwähnten Bestandteilen in der Regel zusätzlich Puffersubstanz geeigneten pH-Werts. Als besonders geeignet erwies sich Trispuffer und GOOD-Puffer. Das getrocknete imprägnierte Trägermaterial wird dann auf das gewünschte Format geschnitten und schließlich gegebenenfalls mit den weiteren, oben erwähnten Bestandteilen zusammengesetzt.

Zur Herstellung von Trockenreagenzien aus löslichen Filmbildnern werden aus den Polymeren Lösungen hergestellt, die so viskos sind, daß sich aus ihnen nach den bekannten Herstellungsverfahren wie Rakeln, Vorhandverfahren, Rollcoating usw. Filme herstellen lassen. In diesen Lösungen werden die Reagenzien, Puffersubstanzen, Hilfsstoffe und Reagenzstabilisatoren eingearbeitet. Die Beschichtungsmassen werden auf Trägerfolien aufgebracht, getrocknet, gegebenenfalls mit weiteren oben erwähnten Bestandteilen zusammengesetzt und die fertigen Filme zu Teststreifen verarbeitet.

Die folgenden Beispiele erläutern die Erfindung weiter in Verbindung mit der Zeichnung. In dieser stellen dar:

- Fig. 1 einen erfindungsgemäßen Teststreifen für die Bestimmung der Quickzeit unter Verwendung von Vollblut,
- Fig. 2 eine Remissionskurve, die unter Verwendung des Teststreifens gemäß Fig. 1 erstellt wurde,
- Fig. 3 eine andere Ausführungsform des erfindungsgemäßen Teststreifens zur Bestimmung von Prothrombin im Vollblut,
- Fig. 4 eine Eichkurve für die Prothrombinbestimmung mit dem Teststreifen von Fig. 3.

**Beispiel 1**

Herstellung eines Teststreifens zur Bestimmung der Quickzeit aus Blut oder Plasma.

Zur Herstellung eines Teststreifens werden 2 Papiere imprägniert:

A Reagenz- und Substratpapier

Man stellt eine Lösung folgender Zusammensetzung her:

| | |
|---|---|
| Tris(HCl | 0,025 mol/l (Puffer) |
| Tos-Gly-Pro-Arg-p-Phenylendiamin | 0,001 mol/l (Substrat) |
| N-Methyl-anthranilsäure, Calciumsalz | 0,03 mol/l (Reagenz) |

Mit 8 ml dieser Lösung wird eine Abfüllung von handelsüblichem Thromboplastin a rekonstituiert und ein Papier geeigneter Dicke und Saugfähigkeit, z. B. Teebeutelpapier, 12 g/m$^2$ Flächengewicht, 0,05 mm Dicke und 50 ml pro m$^2$ Saugvolumen imprägniert. Es erfolgt Trocknung bei 30°C an der Luft und Schneiden in 1 cm breite Streifen.

B Oxidationspapier

Ein gleichartiges Papier wird mit einer Lösung von $K_3(Fe(CN)_6)$, 0,015 mol/l imprägniert und auf 6 mm Breite geschnitten. Das Material wird zu Teststreifen verarbeitet, wie sie in der DE-Offenlegungsschrift 31 30 749 beschrieben sind. Der Aufbau ist in Fig. 1 gezeigt.

Es bedeuten:

1 Trägerfolie
2 Abstandsblock
3 Auftragsmatrix
4 Glasfaservlies (Trennvlies)
5 Glasfaservlies (Transportvlies)
6 Oxidationspapier
7 Reagenz- und Substratpapier
8 Transparente Abdeckfolie. Bringt man 30 µl Citratblut auf das Auftragpapier 3, so durchdringt innerhalb 30 bis 60 Sekunden der Plasmaanteil das gesamte Glasfaservlies 4 und 5, während die Erythrocyten in 4 festgehalten werden. Durch Andrücken der durchsichtigen Folie 8 kommt nun das Plasma mit dem Reagenzpapier und dem Oxidationspapier in Berührung, welche gleichmäßig durchfeuchtet werden. Es bildet sich je nach Aktivität des Plasmas eine Blaufärbung, deren Zeitpunkt des Erscheinens ein Maß für den Quickwert der Probe darstellt. Die Zeit, die vom Andrücken der Folie bis zur Bildung des blauen Farbstoffs verstreicht, ist also ein Maß für den Quickwert der Probe. Das Erscheinen des blauen Farbsignals wird in einem Remissionsspektrometer bei einer Wellenlänge von 565-850 nm gemessen. Zweckmäßigerweise wählt man zur Erkennung des Zeitpunkts eine vorgegebene Remissionsänderung, z. B. 1 oder 2% Remissionsabnahme aus, oder bezieht sich auf eine vorgegebene Menge an gespaltenem Substrat.

**Beispiel 2**

Auf einen Teststreifen nach Beispiel 1 werden 30 µl Citratplasma aufgetragen und nach Andrücken des Reagenzpapiers die Remission in Abhängigkeit der Zeit gemessen. Es ergibt sich die in Fig. 2 dargestellte Remissionskurve. Durch Umrechnung von Remissions-% in die entsprechende Konzentration von gespaltenem Substrat erhält man den zeitlichen Verlauf der Substratspaltung. Legt man zur Ablesung der Reaktionszeit eine Remissionsänderung von 2% gegenüber dem Ausgangswert fest, so ergeben sich nach Fig. 2 45 Sekunden. Dies entspricht einer Substratmenge von 30 µmol, 4,5% der insgesamt vorhandenen Substratmenge.

**Beispiel 3**

Auf Teststreifen nach Beispiel 1 wird abwechselnd nacheinander Citratblut bzw. Citratplasma desselben Spenders aufgetragen. Wenn die Reaktionszeit nach Beispiel 2 berechnet wird, ergibt sich aus mehreren Versuchen im Mittel für Plasma 43,2 Sekunden und für Blut von 44,3 Sekunden, was bei dem für die Methode ermittelten Variationskoeffizienten von 7,7% nicht signifikant voneinander verschieden ist.

**Beispiel 4**

Ein Normalplasmapool von 10 Spendern, hergestellt nach DIN 58939, wird stufenweise mit physiologischer Kochsalzlösung verdünnt. Wenn das unverdünnte Plasma einem Quickwert von 100% der Norm entspricht, so entspricht

die 1:2 Verdünnung 50%, die 1:4 Verdünnung 25% der Norm usw. Die Proben werden auf Teststreifen, die nach Beispiel 1 hergestellt wurden, aufgetragen und die Reaktionszeit nach Beispiel 2 ermittelt. Die Reaktionszeiten werden gegen die reziproken Plasmaverdünnungen aufgetragen. Man erhält eine Gerade, deren Meßpunkte nach linearer Regression mit r = 0,999 korrelieren. Dies bedeutet, daß das Kriterium zur Kalibrierung des Quick-tests mit einem Normalplasmapool gegeben ist, da zwischen der Aktivität der Probe und der Reaktionszeit eine hyperbolische Beziehung besteht, die durch reziproke Auftragung linearisiert werden kann. Ein entsprechender Versuch wird mit Blut und Verdünnung des Bluts eines normalen Spenders durchgeführt und ein Korrelationskoeffizient von r = 0,998 erhalten.

**Beispiel 5**

12 Plasmen gesunder Spender sowie von Spendern, die unter oraler Antikoagulationstherapie stehen, werden auf nach Beispiel 1 hergestellte Teststreifen aufgetragen und die Reaktionszeit bestimmt. An

denselben Plasmen wird der Quickwert mit einem handelsüblichen Quickreagenz (Thromboquant PT) bestimmt. Die Meßwerte beider Testsysteme werden mittels linearer Regression miteinander korreliert. Man erhält die Regressionsgerade y Ø = 15,4 + 0,76 x X und einen Korrelationskoeffizienten r = 0,89.

**Beispiel 6**

Bestimmung von Prothrombin in Blut oder Plasma

Testprinzip:

$$\text{Prothrombin} \xrightarrow[\text{Ca}^{2+},\ \text{Phospholipid}]{\text{F Xa, F V}} \text{Thrombin}$$

$$\text{Tos-Gly-Pro-Arg-NH-Ø-NH}_2 + \text{H}_2\text{O} \xrightarrow{\text{Thrombin}} \text{Tos-Gly-Pro-Arg} + \text{NH}_2\text{-Ø-NH}_2$$

$$\text{NH}_2\text{-Ø-NH}_2 + \text{N-Methylanthranilsäure} + \text{K}_3(\text{Fe(CN)}_6) \longrightarrow \longrightarrow \text{blauer Farbstoff}$$

Den Aufbau des Teststreifens zeigt Fig. 3. In dieser stellen dar:

1 Trägerfolie
2 Abstandsblock
3 Reagenzpapier
4 Glasfaservlies (Trennvlies)
5 Oxidationspapier
6 Auftragsnetz
7 Glasfaservlies (Transportvlies)
8 Substratpapier
9 Transparente Abdeckfolie.

Die auf der linken Seite des Teststreifens verwendeten Vliese haben eine Breite von 0,6 cm; auf der rechten Seite werden Streifen von 1,0 cm Breite verwendet.

Aktivatorvlies (3): Es wird eine Tränklösung folgender Endkonzentration hergestellt:

| | |
|---|---|
| Tris x HCl, 50 mmol/l; pH 8,4; | |
| $CaCl_2$, 5 mmol/l; | |
| Kephalin, 1 g/l; | Cofaktoren der Thrombinbildung |
| Faktor Xa, 1000 U/l; | |
| Faktor V, 100% normal. | |

Mit dieser Tränklösung wird ein Papier geeigneter Dicke und Saugfähigkeit (z. B. Teebeutelpapier)

imprägniert. Es wird bei 30 °C getrocknet und anschließend werden Streifen von 1 cm bzw. 0,6 cm Breite geschnitten.

Oxidationsvlies (5): Es wird eine Tränklösung folgender Zusammensetzung hergestellt:

$K_3$ (Fe(CN)$_6$), 20 mmol/l;
$K_4$ (Fe(CN)$_6$), 20 mmol/l.
Wie beschrieben, wird ein Papier geeigneter Dicke und Saugfähigkeit imprägniert. Es wird bei 30 °C getrocknet und anschließend werden Streifen von 1 cm Breite bzw. 0,6 cm Breite hergestellt.

Substratvlies (8): Es wird eine Tränklösung folgender Zusammensetzung hergestellt:

Tris x HCl, 100 mmol/l, pH 8,1;
N-Methylanthranilsäure, 40 mmol/l;
Substrat (Tos-Gly-Pro-Arg-NH-Ø-$NH_2$), 1 mmol/l.
Wie beschrieben, wird ein Papier geeigneter Dicke und Saugfähigkeit imprägniert. Es wird bei 30 °C getrocknet und anschließend werden Streifen von 1 cm Breite geschnitten.

Durchführung der Prothrombin-Bestimmung:
Es werden 30 µl Citratblut oder Citratplasma, 6,25fach verdünnt, auf das Auftragsnetz 6 aufgetragen. Der Plasma-Anteil der aufgetragenen Probe durchdringt sodann das Oxidations-Vlies 5 (es hat sich als vorteilhaft erwiesen, dieses Vlies bereits an dieser Stelle anzubringen, wo es reaktionsmechanistisch an sich noch nicht benötigt wird), dann durchdringt es das Trennvlies 4 und das Reagenzvlies 3. Anschließend gelangt die Probe mit dem gelösten Reagenz (F V, F Xa, Phospholipid, $Ca^{2+}$) in das Transportvlies 7, wo die Aktivierung von Prothrombin zu Thrombin durch F Xa erfolgt.

Nach 30 Sekunden wird dann die transparente Abdeckfolie 9 und damit die darunter liegenden Vliese (Reagenzvlies 3, Oxidationsvlies 5, Substratvlies 8) auf das Transportvlies 7 angedrückt. Dadurch wird gleichzeitig nochmals F Xa zur Aktivierung von restlichem Prothrombin bereitgestellt und gleichzeitig die Substratreaktion von aktivem Thrombin eingeleitet.

Trägt man diese Konzentrationseinheiten/30 Sekunden gegen den %-Prothrombingehalt der Probe (Normalplasmapool) in einer Verdünnungsreihe auf, dann erhält man eine Eichkurve, an welcher der Prothrombingehalt einer unbekannten Probe abgelesen werden kann (Fig. 4).

**Beispiel 7**

Wie im Beispiel 1 beschrieben, wurde ein Teststreifen hergestellt mit der dort angegebenen Zusammensetzung, jedoch unter Ersatz der N-Methylanthranilsäure als Kupplungskomponente für die Farbbildung durch eine der anderen, in der nachstehenden Tabelle aufgeführten Verbindungen als Kupplungskomponenten. Die Tabelle zeigt die Kupplungskomponenten, die bei der Herstellung der Lösung, mit der das Reagenz- und Substratpapier imprägniert wird, verwendete Konzentration und das Wellenlängenmaximum der bei der Umsetzung mit dem freigesetzten p-Phenylendiamin gebildeten Farbe. Die entsprechenden Werte für N-Methylantranilsäure werden zum Vergleich ebenfalls aufgeführt.

| farbbildende Kupplungs-komponente | MG: | Konzentration in der Lösung | Maximum im Spektrum |
|---|---|---|---|
| N-Methyl-antranilsäure | 151 | 250 mmol/l | 680 nm |
| EST*=(N-Ethyl-N-ß-sulfoethyl-m-toluidin) | 283,32 | 250 mmol/l | 525 nm |
| ETTS** = (N-Ethyl-toluidino-toluolsulfon-säure | 343 | 250 mmol/l | 780 nm |
| Primachin-Diphosphat | 455,35 | 35,1 mmol/l | 460 nm |
| 2,3-Xylenol | 122,17 | 200 mmol/l | 530 nm |
| Diethylmeta-nilsäure | | 20 mg/ml | 715 nm |

* EST wird auch als N-Ethyl 3-methyl-N-(ß-sulfoethyl)-anilin bezeichnet

** ETTS Kaliumsalz

**Beispiel 8**

Test zur Bestimmung der Einphasen-Gerinnungszeit nach Quick (Thromboplastinzeit)

Herstellung der Beschichtungsmasse und des Reagenzfilmes

3,2 mg Tos-Gly-Pro-Arg-p-Phenylendiamin, 52 mg Calcium-N-methyl-anthranilat, 150 mg Polybrene, 300 mg Kaninchenhirnthromboplastin, 10 mg Ficoll werden in 10 ml einer 1,5%igen Lösung von Hydroxypropylcellulose in 0,02 m Hepes-Puffer pH 7,8 eingearbeitet. Die so hergestellte Beschichtungsmasse wird mit einer Naßfilmdicke von 150 μ auf eine transparente Folie beschichtet und bei 35 °C getrocknet.

Herstellen der Oxidationsmatrix

Ein Nylonnetz mit einer Fadenstärke von 30 μ und einer Fadenzahl von 185 Fäden/cm wird mit einer 0,015 molaren Lösung von $K_3[Fe(CN)_6]$ imprägniert und bei 50 °C getrocknet.

Der Reagenzfilm und die Oxidationsmatrix werden zu einem Teststreifen gemäß Figur 1 verarbeitet, wobei lediglich die Reagenzfilmschicht 7 fest an der transparenten Folie 8 anliegt.

Bringt man wie in Beispiel 1 (Text Seite 13) beschrieben Citratblut auf und verfährt wie dort beschrieben, so erhält man bei Heranziehen der Zeit, die für eine Remissionsabnahme von 2 % benötigt wird, als Meßgröße folgende Meßwerte:

| % Quick | Sekunden bis zu einer Abnahme um 2 % Remission |
|---|---|
| 100 % | 51,5 |
| 50 % | 56,4 |
| 35 % | 64,3 |
| 25 % | 75,0 |
| 12,4 % | 107 |
| 10 % | 121 |

**Ansprüche**

1. Trockenreagenz zur Durchführung von Quick-Tests, PTT-Tests, zur Bestimmung von Prothrombin, Faktor X, Faktor VIII, Faktor VII und Faktor IX, **gekennzeichnet durch** ein Trägermaterial, welches ein chromophores Substrat einer Protease des Blutgerinnungs-Systems, mit mindestens einem Faktor und/oder Cofaktor des Blutgerinnungs-Systems und mit Puffersubstanz enthält.

2. Trockenreagenz nach Anspruch 1,
**dadurch gekennzeichnet,** daß es ein zweites Trägermaterial mit einem Oxidationsmittel enthält und das erste Trägermaterial ein mit dem Chromophor des chromophoren Substrats in Gegenwart des Oxidationsmittels des zweiten Trägermaterials farbbildendes Anilin- oder Phenolderivat enthält.

3. Trockenreagenz nach Anspruch 1 oder 2,
**dadurch gekennzeichnet,** daß es für die Durchführung des Quick-Tests Thromboplastin, ein chromophores Thrombinsubstrat und $Ca^{2+}$-Ionen enthält.

4. Trockenreagenz nach Anspruch 1 oder 2,
**dadurch gekennzeichnet,** daß es für die Bestimmung von Prothrombin Faktor Xa und als Cofaktoren Faktor V, $Ca^{2+}$ und Phospholipid enthält.

5. Trockenreagenz nach Anspruch 1 oder 2,
**dadurch gekennzeichnet,** daß es für die Durchführung des partielle Thromboplastinzeit(PTT)-Tests partielles Thromboplastin und Kontaktaktivatoren, ein chromophores Thrombinsubstrat und $Ca^{2+}$-Ionen enthält.

6. Trockenreagenz nach Anspruch 5,
**dadurch gekennzeichnet,** daß es Ellagsäure, Phospholipid und $Ca^{2+}$ enthält.

7. Trockenreagenz nach Anspruch 1 oder 2,
**dadurch gekennzeichnet,** daß es für die Bestimmung von Faktor X das Gift der Russel's Viper oder Faktor X Aktivator aus diesem Gift der , $Ca^{2+}$ und ein chromophores Faktor Xa Substrat enthält.

8. Trockenreagenz nach Anspruch 1 oder 2,
**dadurch gekennzeichnet,** daß es für die Bestimmung von Faktor VIII Faktor IXa, Thrombin, $Ca^{2+}$ , Phospholipid und ein chromophores Faktor Xa- oder Thrombinsubstrat enthält.

9. Trockenreagenz nach Anspruch 1 oder 2,
**dadurch gekennzeichnet,** daß es für die Bestimmung von Faktor VII Thromboplastin, $Ca^{2+}$ und ein chromophores Faktor Xa Substrat enthält.

10. Trockenreagenz nach Anspruch 1 oder 2,

**dadurch gekennzeichnet,** daß es für die Bestimmung von Faktor IX aktivierte Kontaktfaktoren, Faktor XIa, $Ca^{2+}$ und entweder ein chromophores Faktor IXa Substrat oder Phospholipid, Faktor VIII, Thrombin und ein chromophores Faktor Xa Substrat enthält.

**11.** Trockenreagenz nach einem der vorhergehenden Ansprüche, **dadurch gekennzeichnet,** daß es als chromophores Substrat eine Verbindung der allgemeinen Formel I enthält

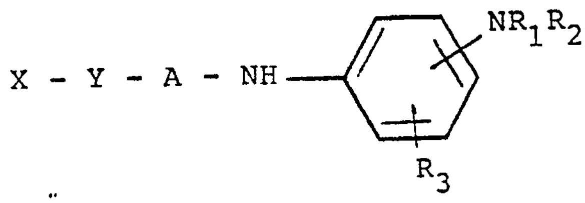

in der

| | |
|---|---|
| A | die Aminosäure Arginin oder Lysin, |
| X | eine N-terminale Aminosäure-Schutzgruppe, |
| Y | eine Einfachbindung oder eine aus 1 bis 3 Aminosäuren bestehende Kette, |
| $NR_1R_2$ | eine in o- oder p-Stellung stehende Gruppe mit $R_1$ und $R_2$ unabhängig voneinander jeweils Wasserstoff oder Alkyl mit 1 bis 3 C-Atomen oder $NO_2$ und |
| $R_3$ | ein Wasserstoffatom, eine Carboxylester- oder Carboxylamidogruppe, ein Halogenatom, eine Nitrogruppe oder eine Alkylgruppe mit 1 bis 3 C-Atomen bedeutet. |

**12.** Trockenreagenz nach Anspruch 2, **dadurch gekennzeichnet,** daß es als farbbildendes Anilin- oder Phenolderivat N-Methylanthranilsäure, Dimethylanthranilsäure, N-Ethyl-n-(3'-sulfobenzol)-anilin oder 2,3-Xylenol enthält.

**13.** Trockenreagenz nach den Ansprüchen 1 und 11, **dadurch gekennzeichnet,** daß das Trägermaterial als chromophores Substrat ein Tos-Gly-Pro-Arg-Derivat enthält.

**14.** Trockenreagenz nach Anspruch 13, **dadurch gekennzeichnet,** daß das erste Trägermaterial als chromophores Substrat Tos-Gly-Pro-Arg-p-Phenylendiamin, als farbbildendes Anilinderivat N-Methylanthranilsäure und das zweite saugfähige Trägermaterial als Oxidationsmittel $K_3(Fe(CN)_6)$ enthält.

**15.** Trockenreagenz nach einem der vorhergehenden Ansprüche, **dadurch gekennzeichnet,** daß das erste Trägermaterial ein saugfähiges Trägermaterial ist.

**16.** Trockenreagenz nach einem der vorhergehenden Ansprüche, **dadurch gekennzeichnet,** daß es zur Bestimmung mit Vollblut zusätzlich ein drittes saugfähiges Trägermaterial für das Aufbringen der Blutprobe und gegebenenfalls ein Faservlies als Trenn- und Transportmatrix, welches zwischen dem dritten und dem ersten Trägermaterial und gegebenenfalls zwischen dem ersten und zweiten Trägermaterial angeordnet ist, enthält.

**17.** Trockenreagenz nach Anspruch 16, **dadurch gekennzeichnet,** daß es mindestens ein Glasfaservlies enthält.

**18.** Trockenreagenz nach einem der vorhergehenden Ansprüche, **dadurch gekennzeichnet,** daß es eine Trägerfolie enthält.

**19.** Trockenreagenz nach einem der vorhergehenden Ansprüche, **dadurch gekennzeichnet,** daß es eine Abdeckfolie enthält.

**20.** Trockenreagenz nach einem der vorhergehenden Ansprüche, **dadurch gekennzeichnet,** daß es eine Auftragsfolie enthält.

**21.** Trockenreagenz nach einem der vorhergehenden Ansprüche, **dadurch gekennzeichnet,** daß es wenigstens einen Abstandsblock enthält.

## Claims

**1.** Dry reagent for the carrying out of Quick tests, PTT tests, for the determination of prothrombin, Factor X, Factor VIII, Factor VII and Factor IX, characterised by a carrier material which contains a chromophoric substrate of a protease of the blood coagulation system, with at least one factor and/or co-factor of the blood coagulation system and with buffer.

**2.** Dry reagent according to claim 1, characterised in that it contains a second carrier material with an oxidation agent and the first carrier material contains an aniline or phenol derivative forming a colour with the chromophore of the chromophoric substrate in the presence of the oxidation agent of the second carrier material.

**3.** Dry reagent according to claim 1 or 2, characterised in that, for the carrying out of the Quick test, it contains thromboplastin, a chromophoric thrombin substrate and $Ca^{2+}$-ions.

**4.** Dry reagent according to claim 1 or 2, characterised in that, for the determination of prothrombin, it contains Factor Xa and, as co-factors, Factor V, $Ca^{2+}$ and phospholipid.

**5.** Dry reagent according to claim 1 or 2, characterised in that, for the carrying out of the partial prothromboplastin time (PTT) test, it contains partial thromboplastin and contact activators, a chromophoric thrombin substrate and $Ca^{2+}$-ions.

**6.** Dry reagent according to claim 5, characterised in that it contains ellagic acid, phospholipid and $Ca^{2+}$.

**7.** Dry reagent according to claim 1 or 2, characterised in that, for the determination of Factor X, it contains the venom of Russel's viper or Factor X activator from this venom, $Ca^{2+}$ and a chromophoric Factor Xa substrate.

**8.** Dry reagent according to claim 1 or 2, characterised in that, for the determination of Factor VIII, it contains Factor IXa, thrombin, $Ca^{2+}$, phospholipid and a chromophoric Factor Xa or thrombin substrate.

**9.** Dry reagent according to claim 1 or 2, characterised in that, for the determination of Factor VII, it contains thromboplastin, $Ca^{2+}$ and a chromophoric Factor Xa substrate.

**10.** Dry reagent according to claim 1 or 2, characterised in that, for the determination of Factor IX, it contains activated contact factors, Factor XIa, $Ca^{2+}$ and either a chromophoric Factor IXa substrate or phospholipid, Factor VIII, thrombin and a chromophoric Factor Xa substrate.

**11.** Dry reagent according to one of the preceding claims, characterised in that, as chromophoric substrate, it contains a compound of the general formula I

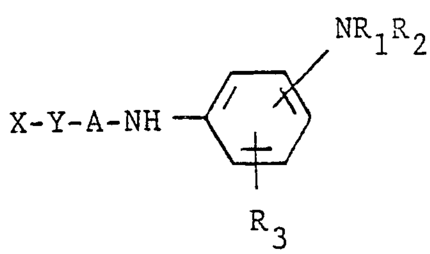

in which A signifies the amino acid arginine or lysine, X an N-terminal amino acid protective group, Y a single bond or a chain consisting of 1 to 3 amino acids, $NR_1R_2$ a group standing in the o - or p - position with $R_1$ and $R_2$, independently of one another, each hydrogen or alkyl with 1 to 3 C-atoms or $NO_2$ and $R_3$ a hydrogen atom, a carboxyl ester or carboxylamido group, a halogen atom, a nitro group

or an alkyl group with 1 to 3 C-atoms.

**12.** Dry reagent according to claim 2, characterised in that, as colour-forming aniline or phenol derivative, it contains N-methylanthranilic acid, dimethylanthranilic acid, N-ethyl-N-(3'-sulphobenzene)-aniline or 2,3-xylenol.

**13.** Dry reagent according to claims 1 and 11, characterised in that the carrier material contains a Tos-Gly-Pro-Arg derivative as chromophoric substrate.

**14.** Dry reagent according to claim 13, characterised in that the first carrier material contains, as chromophoric substrate, Tos-Gly-Pro-Arg-p -phenylenediamine, as colour-forming aniline derivative N-methyanthranilic acid and the second absorbent carrier material $K_3(Fe(CN)_6)$ as oxidation agent.

**15.** Dry reagent according to one of the preceding claims, characterised in that the first carrier material is an absorbent carrier material.

**16.** Dry reagent according to one of the preceding claims, characterised in that, for the determination with whole blood, it additionally contains a third absorbent material for the application of the blood sample and possibly a fibre fleece as separating and transport matrix which is arranged between the third and the first carrier material and possibly between the first and second carrier material.

**17.** Dry reagent according to claim 16, characterised in that it contains at least one glass fibre fleece.

**18.** Dry reagent according to one of the preceding claims, characterised in that it contains a carrier film.

**19.** Dry reagent according to one of the preceding claims, characterised in that it contains a covering film.

**20.** Dry reagent according to one of the preceding claims, characterised in that it contains an application film.

**21.** Dry reagent according to one of the preceding claims, characterised in that it contains at least one distance piece.

## Revendications

**1.** Réactif sec pour la mise en oeuvre des essais de Quick, des essais PTT, pour la détermination de la prothrombine, du facteur X, du facteur VIII, du facteur VII et du facteur IX, caractérisé par un matériau support qui contient un substrat chromophore d'une protéase du système de coagulation du sang, avec au moins un facteur et/ou co-facteur du système de coagulation du sang et avec de la substance tampon.

**2.** Réactif sec selon la revendication 1, caractérisé en ce qu'il contient un deuxième matériau support avec un agent d'oxydation et en ce que le premier matériau support contient un dérivé d'aniline ou de phénol formant une coloration avec le chromophore du substrat chromophore en présence de l'agent d'oxydation du deuxième matériau support.

**3.** Réactif sec selon la revendication 1 ou 2, caractérisé en ce que, pour la mise en oeuvre de l'essai Quick, il contient de la thromboplastine, un substrat chromophore de la thrombine et des ions $Ca^{2+}$.

**4.** Réactif sec selon la revendication 1 ou 2, caractérisé en ce que, pour la détermination de la prothrombine, il contient du facteur Xa et comme co-facteurs du facteur V, $Ca^{2+}$ et du phospholipide.

**5.** Réactif sec selon la revendication 1 ou 2, caractérisé en ce que, pour la mise en oeuvre de l'essai du temps partiel de thromboplastine (PTT), il contient de la thromboplastine partielle et des activateurs contact, un substrat de thrombine chromophore et des ions $Ca^{2+}$.

**6.** Réactif sec selon la revendication 5, caractérisé en ce qu'il contient de l'acide ellagique, du phospholi-

pide et $Ca^{2+}$.

**7.** Réactif sec selon la revendication 1 ou 2, caractérisé en ce que, pour la détermination du facteur X, il contient le poison de la vipère de Russel ou de l'activateur de facteur X provenant de ce poison, $Ca^{2+}$ et un substrat chromophore du facteur Xa.

**8.** Réactif sec selon la revendication 1 ou 2, caractérisé en ce que, pour la détermination du facteur VIII, il contient du facteur IXa, de la thrombine, $Ca^{2+}$, du phospholipide et un substrat chromophore du facteur Xa ou de la thrombine.

**9.** Réactif sec selon la revendication 1 ou 2, caractérisé en ce que, pour la détermination du facteur VII, il contient de la thromboplastine, $Ca^{2+}$ et un substrat chromophore du facteur Xa.

**10.** Réactif sec selon la revendication 1 ou 2, caractérisé en ce que, pour la détermination du facteur IX, il contient des facteurs contact activés, du facteur XIa, $Ca^{2+}$ et soit un substrat chromophore du facteur IXa, soit du phospholipide, du facteur VIII, de la thrombine et un substrat chromophore du facteur Xa.

**11.** Réactif sec selon l'une des revendications précédentes, caractérisé en ce qu'il contient, comme substrat chromophore, un composé de formule générale I

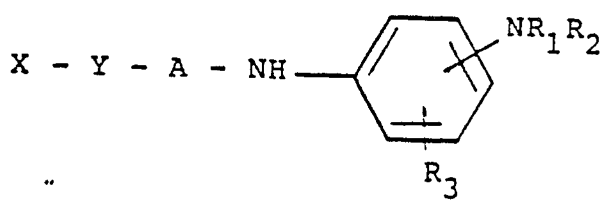

dans laquelle

| | |
|---|---|
| A | représente l'aminoacide arginine ou lysine, |
| X | représente un groupe protecteur d'aminoacide N-terminal, |
| Y | représente une liaison simple ou une chaîne constituée de 1 à 3 aminoacides, |
| $NR_1R_2$ | représente un groupe se trouvant en position o ou p, $R_1$ et $R_2$ étant, indépendamment l'un de l'autre, chacun hydrogène ou alcoyle avec 1 à 3 atomes de C ou $NO_2$ et |
| $R_3$ | représente un atome d'hydrogène, un groupe carboxylester ou carboxylamido, un atome d'halogène, un groupe nitro ou un groupe alcoyle avec 1 à 3 atomes de C. |

**12.** Réactif sec selon la revendication 2, caractérisé en ce qu'il contient, en tant que dérivé d'aniline ou de phénol formant une coloration, de l'acide N-méthylanthranilique, de l'acide diméthylanthranilique, de la N-éthyl-n-(3'-sulfobenzène)-aniline ou du 2,3-xylénol.

**13.** Réactif sec selon les revendications 1 et 11, caractérisé en ce que le matériau support contient, comme substrat chromophore, un dérivé de Tos-Gly-Pro-Arg.

**14.** Réactif sec selon la revendication 13, caractérisé en ce que le premier matériau support contient comme substrat chromophore de la Tos-Gly-Pro-Arg-p-phénylènediamine, comme dérivé d'aniline formant une coloration de l'acide N-méthylanthranilique et le deuxième matériau support absorbant contient comme agent d'oxydation $K_3(Fe(CN)_6)$.

**15.** Réactif sec selon l'une des revendications précédentes, caractérisé en ce que le premier matériau support est un matériau support absorbant.

**16.** Réactif sec selon l'une des revendications précédentes, caractérisé en ce que, pour la détermination du sang complet, il contient en outre un troisième matériau support absorbant pour le dépôt de l'échantillon de sang et éventuellement un non-tissé de fibres comme matrice de séparation et de transport, laquelle est placée entre le troisième et le premier matériaux supports et éventuellement entre le premier et le deuxième matériaux supports.

17. Réactif sec selon la revendication 16, caractérisé en ce qu'il ccntient au moins un non-tissé de fibres de verre.

18. Réactif sec selon l'une des revendications précédentes, caractérisé en ce qu'il contient une feuille support.

19. Réactif sec selon l'une des revendications précédentes, caractérisé en ce qu'il contient une feuille de recouvrement.

20. Réactif sec selon l'une des revendications précédentes, caractérisé en ce qu'il contient une feuille de dépôt.

21. Réactif sec selon l'une des revendications précédentes, caractérisé en ce qu'il contient au moins un bloc d'écartement.

FIG. 1

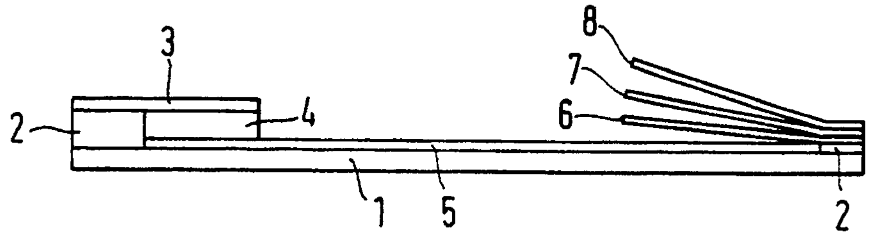


FIG 3

PROTHROMBIN

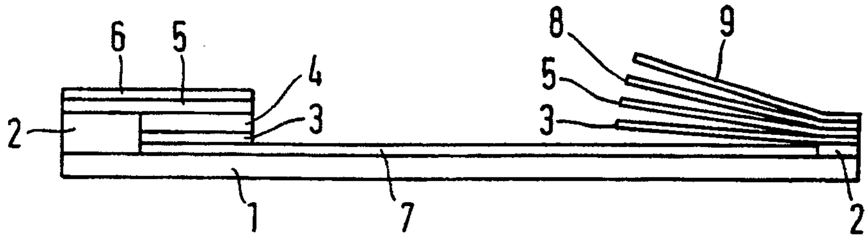

FIG. 2

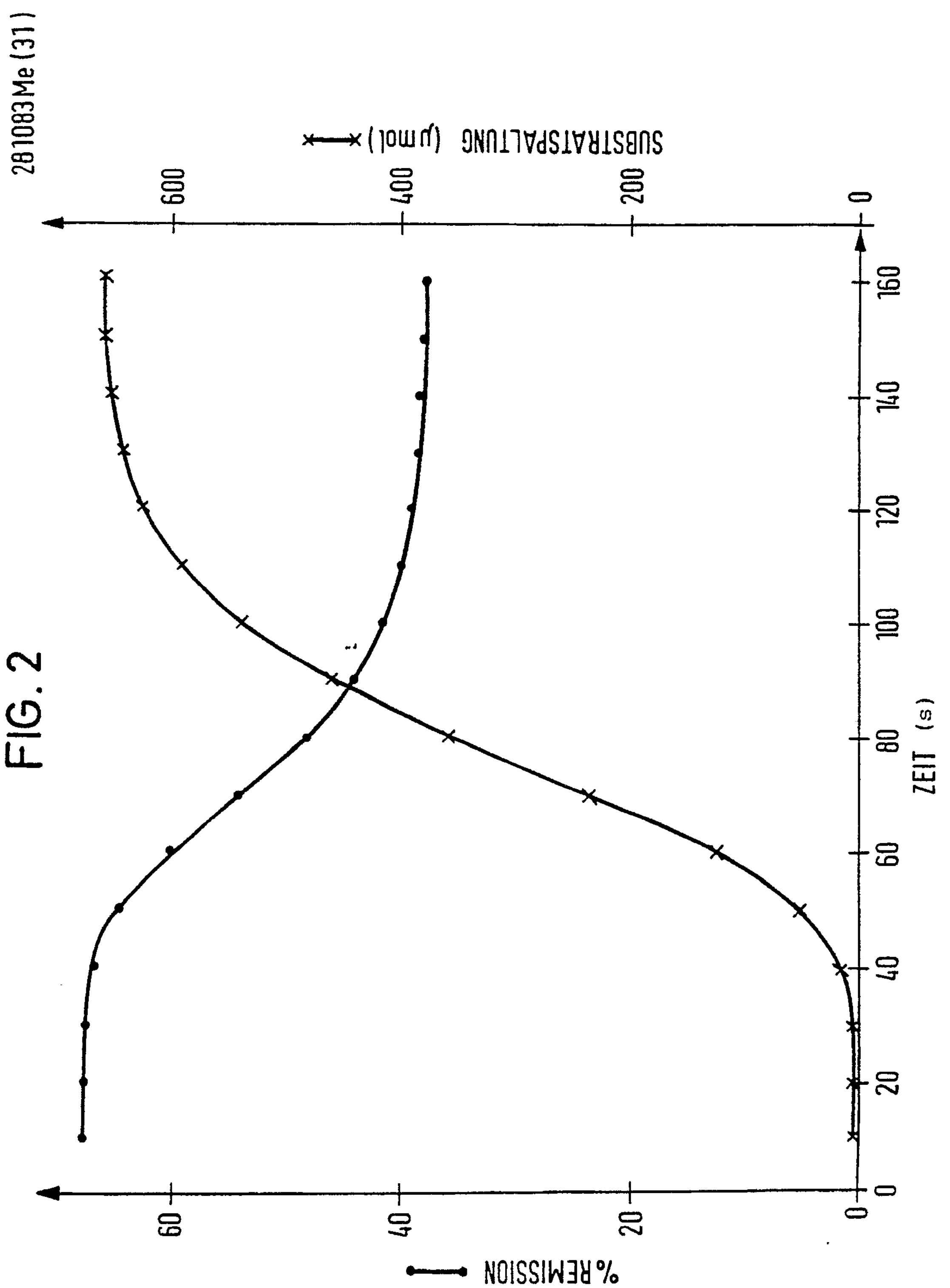
281083 Me (31)
SUBSTRATSPALTUNG (µmol)
600
400
200
0
%REMISSION
60
40
20
0
0
20
40
60
80
100
120
140
160
ZEIT (s)

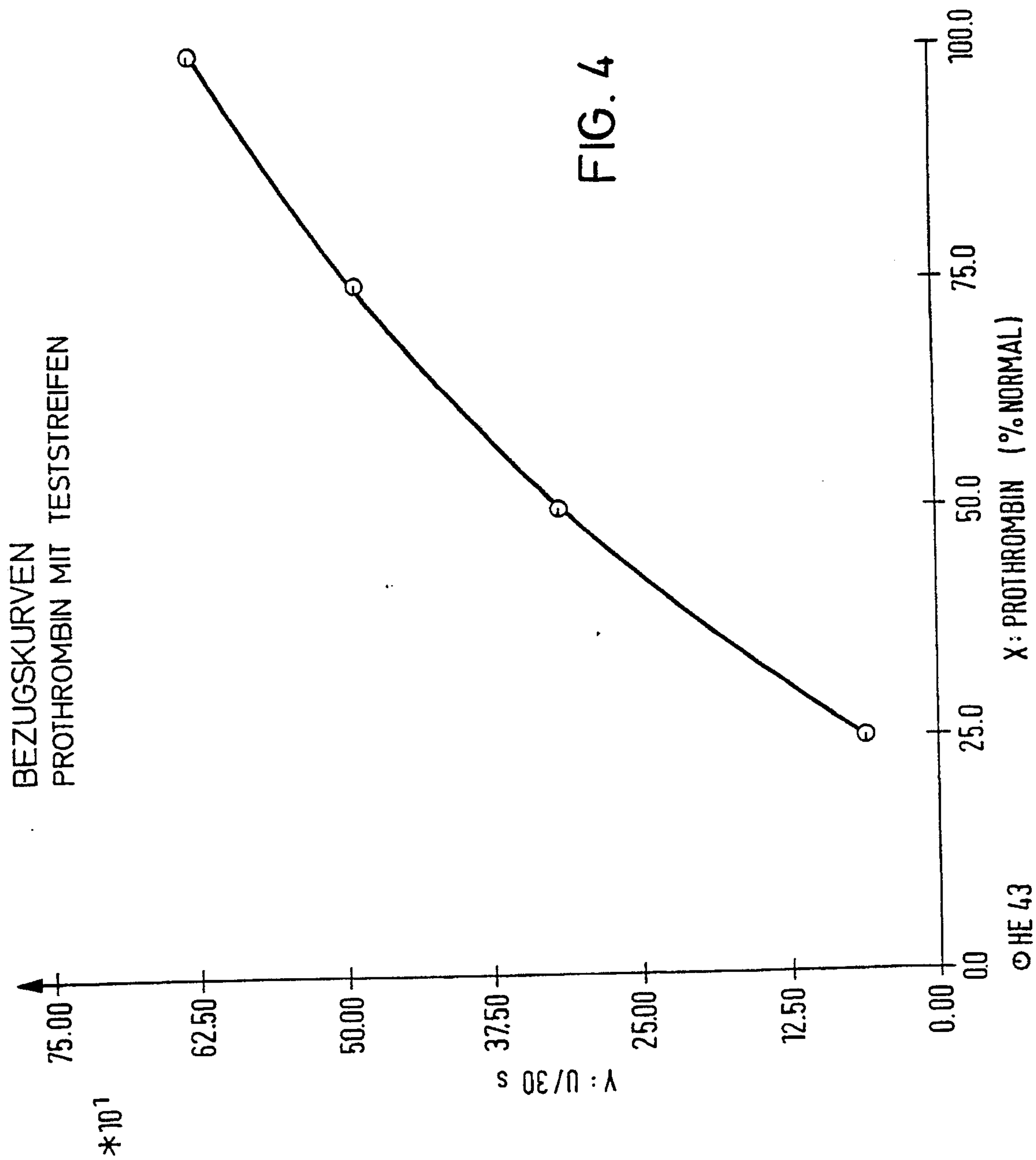
BEZUGSKURVEN
PROTHROMBIN MIT TESTSTREIFEN
Y : U/30 s
*10¹
75.00
62.50
50.00
37.50
25.00
12.50
0.00
0.0
25.0
50.0
75.0
100.0
X : PROTHROMBIN (% NORMAL)
HE 43

FIG. 4